# EUROPÄISCHE PATENTSCHRIFT

(11) **EP 0 752 095 B1**
(45) Veröffentlichungstag und Bekanntmachung des Hinweises auf die Patenterteilung: **23.06.1999**
(21) Anmeldenummer: 95907570.6
(22) Anmeldetag: 20.01.1995
(51) Int. Cl.: G01M 3/20

(54) **TESTGAS-LECKSUCHGERÄT**
TEST GAS LEAK DETECTOR
APPAREIL DE DETECTION DE FUITE A L'AIDE DE GAZ TEMOIN

(30) Priorität: 24.03.1994 DE 9405028 U
(43) Veröffentlichungstag der Anmeldung: 08.01.1997
(73) Patentinhaber: LEYBOLD AKTIENGESELLSCHAFT, 50968 Köln (DE)
(72) Erfinder: BÖHM, Thomas, D-50933 Köln (DE); DÖBLER, Ulrich, D-42929 Wermelskirchen (DE)
(74) Vertreter: Leineweber, Jürgen, Dipl.-Phys.
(86) Internationale Anmeldenummer: EP9500202
(87) Internationale Veröffentlichungsnummer: WO9525947

(56) Entgegenhaltungen:
- EP-A- 0 158 295
- EP-A- 0 268 777
- DE-A- 4 228 313

## Beschreibung

Die Erfindung bezieht sich auf ein für die Durchführung der Testgas-Lecksuche geeignetes Lecksuchgerät mit einem Testgasanschluß, mit einem Testgasdetektor, mit einer Gasförderpumpe und mit einer Testgasleitung, die sich zwischen dem Testgasanschluß und der Gasförderpumpe erstreckt und die über einen absperrbaren Abzweig mit dem Einlaß des Testgasdetektors in Verbindung steht. Bei offenem Abzweig - Meßbetrieb - gelangt ein Teil des durch die Testgasleitung strömenden Gases, das für den Fall des Vorhandenseins eines Lecks Testgas enthält, über die Abzweigleitung in den Testgasdetektor und wird dort registriert.

Bei Lecksuchgeräten dieser Art ist die Ansprechzeit, das heißt, die Zeit, die vom Zeitpunkt des Eintritts von Testgas in den Testgasanschluß bis zum Zeitpunkt der Registrierung des Testgases vergeht, relativ lang, insbesondere bei höheren Drücken.

Der vorliegenden Erfindung liegt die Aufgabe zugrunde, bei einem Lecksuchgerät der eingangs erwähnten Art die Ansprechzeit zu verkürzen.

Erfindungsgemäß wird diese Aufgabe durch die Merkmale des Patentanspruchs 1 gelöst.

Durch die vorgeschlagenen Maßnahmen wird erreicht, daß bei der Betriebsart "Messen" im wesentlichen das gesamte in der Testgasleitung strömende Gas unmittelbar in den Testgasdetektor gelangt. Dadurch wird die Ansprechzeit stark verkürzt, bei höheren Drücken sogar um mehr als den Faktor 10.

Umfaßt der Testgasdetektor ein Massenspektrometer und eine dem Massenspektrometer vorgeschaltete, entgegen ihrer Förderrichtung vom Testgas durchströmte Hochvakuumpumpe, vorzugsweise Turbomolekularvakuumpumpe, dann wird durch die Erfindung erreicht, daß das durch die Testgasleitung strömende Gas und damit das darin gegebenenfalls vorhandene Testgas vollständig und unmittelbar in den Auslaßbereich der Hochvakuumpumpe gelangt, so daß die gewünschte Verkürzung der Ansprechzeit erreicht werden kann.

Weitere Vorteile und Einzelheiten der Erfindung sollen anhand von in den Figuren 1 bis 6 dargestellten Ausführungsbeispielen erläutert werden. Es zeigen
- Figuren 1 bis 4 schematisch dargestellte Lecksuchgeräte nach der Erfindung sowie
- Figuren 5 und 6 ein Ausführungsbeispiel für die Gestaltung der erfindungsgemäßen Verbindung zwischen Testgasleitung und einer Hochvakuumpumpe.

In den Figuren sind das Lecksuchgerät generell mit 1, der Testgasanschluß mit 2, der Testgasdetektor mit 3, die Gasförderpumpe oder Vorvakuumpumpe mit 4 und die sich zwischen Testgasanschluß 2 und Vorvakuumpumpe 4 erstreckende Testgasleitung mit 5 bezeichnet. Die Testgasleitung 5 ist mit dem Einlaßbereich 6 des Testgasdetektors 3 über zwei Leitungsabschnitte 7 und 8 verbunden.

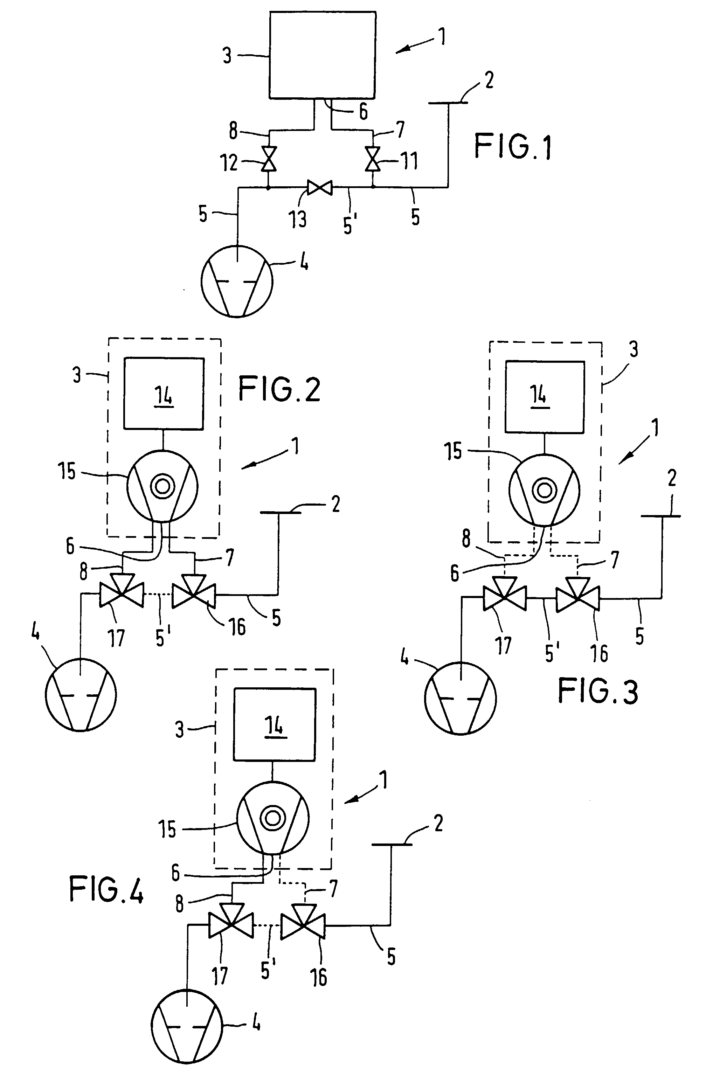

Beim Ausführungsbeispiel nach Figur 1 sind jedes der Leitungsabschnitte 7 und 8 sowie das Zwischenstück 5' der Testgasleitung 5 zwischen den Anschlüssen der Leitungsabschnitte 7 und 8 an die Testgasleitung 5 mit jeweils einem 2/2-Wegeventil 11, 12 bzw. 13 ausgerüstet. Beim Meßbetrieb sind die Ventile 11 und 12 geöffnet, das Ventil 13 geschlossen. Das in den Testgaseinlaß 2 gelangende Gas durchströmt in seiner Gesamtheit unmittelbar den Einlaßbereich 6 des Testgasdetektors 3. Bei geschlossenen Ventilen 11, 12 und offenem Ventil 13 - Pumpbetrieb - besteht die Möglichkeit, einen mit dem Testgasanschluß 2 verbundenen Prüfling oder Rezipienten zu evakuieren. Beim "Standby"-Betrieb sind die Ventile 11, 13 geschlossen und das Ventil 12 geschlossen.

Bei den Ausführungsbeispielen nach den Figuren 2 bis 4 umfaßt der Testgasdetektor 3 ein Massenspektrometer 14 und eine Hochvakuumpumpe 15, vorzugsweise eine Turbomolekularvakuumpumpe. Die Hochvakuumpumpe 15 dient der Aufrechterhaltung des für den Betrieb des Massenspektrometers 14 erforderlichen Vakuums (ca. 10⁻⁴ mbar). Bei dieser Ausführungsform durchströmt das Testgas - leichtes Gas, in der Regel Helium - die Hochvakuumpumpe entgegen ihrer Förderrichtung. Der Einlaß 6 des Testgasdetektors 3 ist deshalb gleichzeitig der Auslaß der Hochvakuumpumpe 15.

Anstelle von drei 2/2-Wegeventilen 11, 12, 13 in den Leitungsabschnitten 7, 8 sind zwei 2/3-Wegeventile 16, 17 vorgesehen, die gleichzeitig die Anschlüsse der Leitungsabschnitte 7, 8 an der Testgasleitung 5 bilden. In den Figuren 2 bis 4 sind die Leitungsabschnitte 7, 8 und das Zwischenstück 5' ausgezogen (offen) oder gestrichelt (abgesperrt) dargestellt. Die jeweilige Öffnung bzw. Sperrung wird durch entsprechende Stellungen der Ventile 16, 17 erreicht. Figur 2 zeigt den Meßbetrieb, Figur 3 den Pumpbetrieb und Figur 4 den Standby-Betrieb.

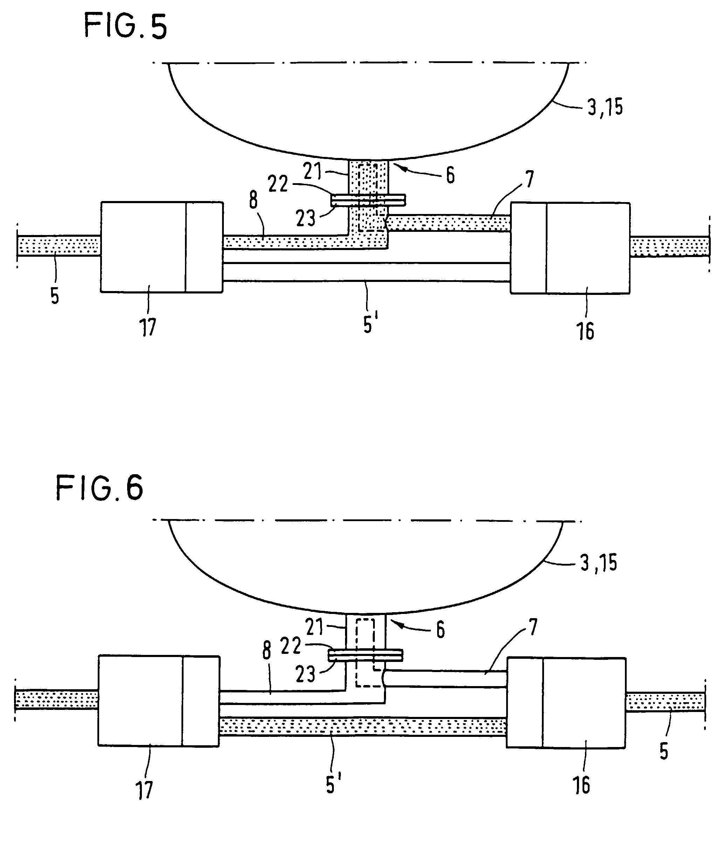

Die Figuren 5 (Meßbetrieb) und 6 (Pumpbetrieb) lassen eine zweckmäßige Gestaltung des Einlaßbereiches 6 des Testgasdetektors 3 (bzw. des Auslasses 6 der Hochvakuumpumpe 15) erkennen. Bestandteil des Einlasses 6 des Testgasdetektors 3 (bzw. des Auslasses 6 der Hochvakuumpumpe 15) ist ein Anschlußstutzen 21 mit einem Flansch 22. An diesem Flansch 22 ist der Leitungsabschnitt 8 - ebenfalls mit einem Flansch 23 ausgerüstet - angeschlossen. Zumindest im flanschnahen Bereich weist die Leitung 8 einen größeren Durchmesser als der Leitungsabschnitt 7 auf. In diesem Bereich ist der Leitungsabschnitt 7 in die Leitung 8 hineingeführt. Er durchsetzt den flanschnahen Bereich der Leitung 8 und endet in Höhe der Mündung der Leitung 8 in den Testgasdetektor 3 bzw. die Hochvakuumpumpe 15. Durch diese Lösung ist sichergestellt, daß das gesamte, die Testgasleitung 5 durchströmende Gas in den Einlaß 6 des Testgasdetektors 3 bzw. den Auslaß 6 der Hochvakuumpumpe 15 gelangt.

## Patentansprüche

1. Lecksuchgerät (1), für die Durchführung der Testgas-Lecksuche, mit einem Testgasanschluß (2), einem Testgasdetektor (3), einer Gasförderpumpe (4) und einer Testgasleitung (5), die sich zwischen dem Testgasanschluß (2) und der Gasförderpumpe (4) erstreckt und die über einen absperrbaren Abzweig mit dem Einlaß (6) des Testgasdetektors (3) in Verbindung steht, dadurch gekennzeichnet, daß eine der Verbindung der Testgasleitung (5) mit dem Einlaßbereich (6) des Testgasdetektors dienende Leitungsführung (7, 8) so gewählt ist, daß im Meßbetrieb im wesentlichen das gesamte in der Testgasleitung (5) strömende Gas in den Einlaßbereich (6) des Testgasdetektors (3) gelangt.

2. Lecksuchgerät nach Anspruch 1, dadurch gekennzeichnet, daß die Testgasleitung (5) über zwei absperrbare Leitungsabschnitte (7, 8) mit dem Einlaß (6) des Testgasdetektors (3) verbunden ist und daß das Zwischenstück (5') der Testgasleitung (5) zwischen den Anschlußstellen der zum Testgasdetektor (3) führenden Leitungsabschnitte (7, 8) ebenfalls absperrbar ist.

3. Lecksuchgerät nach Anspruch 2, dadurch gekennzeichnet, daß die beiden Leitungsabschnitte (7, 8) und das Zwischenstück (5') der Testgasleitung (5) jeweils mit einem 2/2-Wegeventil ausgerüstet sind.

4. Lecksuchgerät nach Anspruch 2, dadurch gekennzeichnet, daß sich im Bereich der Anschlußstellen der beiden Leitungsabschnitte (7, 8) an die Testgasleitung (5) je ein 3/2-Wegeventil (16 bzw. 17) befindet.

5. Lecksuchgerät nach einem der vorhergehenden Ansprüche, dadurch gekennzeichnet, daß die beiden Leitungsabschnitte (7, 8) im Bereich des Einlasses (6) des Testgasdetektors (3) unterschiedliche Durchmesser haben und konzentrisch zueinander geführt sind.

6. Lecksuchgerät nach Anspruch 5, dadurch gekennzeichnet, daß der Testgasdetektor (3) einen Anschlußstutzen (21) mit einem Flansch (22) aufweist, daß die Abzweigleitung (7 oder 8) mit dem größeren Durchmesser über einen Flansch (23) mit dem Anschlußstutzen (21) verbunden ist und daß die Abzweigleitung (7 oder 8) mit dem kleineren Durchmesser konzentrisch den Anschlußstutzen (21) durchsetzt.

7. Lecksuchgerät nach einem der vorhergehenden Ansprüche, dadurch gekennzeichnet, daß der Testgasdetektor (3) ein Massenspektrometer (14) und eine vom Testgas entgegen ihrer Förderrichtung durchströmte Hochvakuumpumpe (15) umfaßt und daß der Auslaß der Hochvakuumpumpe (15) den Einlaß (6) des Massenspektrometers (3) bildet.

## Claims

1. A leak locator (1) for carrying out the test gas leak search, with a test gas connection (2), a test gas detector (3), a gas conveying pump (4) and a test gas line (5), which extends between the test gas connection (2) and the gas conveying pump (4) and is connected via a closable branch to the inlet (6) of the test gas detector (3), characterised in that a line run (7, 8) used to connect the test gas line (5) to the inlet region (6) of the test gas detector is selected in such a manner that, during measuring operation, substantially the entire gas flowing in the test gas line (5) enters the inlet region (6) of the test gas detector (3).

2. A leak locator according to claim 1, characterised in that the test gas line (5) is connected via two closable line sections (7, 8) to the inlet (6) of the test gas detector (3) and the intermediate section (5') of the test gas line (5) between the connection sites of the lines sections (7, 8) leading to the test gas detector (3) can also be closed.

3. A leak locator according to claim 2, characterised in that the two line sections (7, 8) and the intermediate section (5') of the test gas line (5) are each provided with a 2/2-way valve.

4. A leak locator according to claim 2, characterised in that a 3/2-way valve (16 or 17) is disposed in the region of each of the connection sites of the two line sections (7, 8) to the test gas line (5).

5. A leak locator according to one of the preceding claims, characterised in that, in the region of the inlet (6) of the test gas detector (3), the two line sections (7, 8) have different diameters and are guided concentric to one another.

6. A leak locator according to claim 5, characterised in that the test gas detector (3) comprises a connection socket (21) with a flange (22), the branch line (7 or 8) with the larger diameter is connected via a flange (23) to the connection socket (21) and the branch line (7 or 8) with the smaller diameter penetrates the connection socket (21) concentrically.

7. A leak locator according to one of the preceding claims, characterised in that the test gas detector (3) comprises a mass spectrometer (14) and a high vacuum pump (15), through which test gas flows in the opposite direction to the conveying direction of said pump, and the outlet of the high vacuum pump (15) forms the inlet (6) of the mass spectrometer (3).

## Revendications

1. Détecteur de fuite (1) en vue de la mise en oeuvre de la détection d'une fuite d'un gaz de référence, équipé d'un raccord de gaz de référence (2), d'un détecteur de gaz de référence (3), d'une pompe de circulation de gaz (4) et d'une canalisation de gaz de référence (5), qui s'étend entre le raccord de gaz de référence (2) et la pompe de circulation de gaz (4) et qui se trouve en liaison, par l'intermédiaire d'une dérivation pouvant être fermée, avec l'admission (6) du détecteur de gaz de référence (3), caractérisé en ce qu'un tracé de canalisation (7, 8) servant à la liaison de la canalisation de gaz de référence (5) avec l'admission (6) du détecteur de gaz de référence est choisi de sorte que durant la mesure, sensiblement la totalité du gaz circulant dans la canalisation de gaz de référence (5) parvient à l'admission (6) du détecteur de gaz de référence (3).

2. Détecteur de fuite selon la revendication 1, caractérisé en ce que la canalisation de gaz de référence (5) est reliée, par l'intermédiaire de deux tronçons de canalisation (7, 8) pouvant être fermés, avec l'admission (6) du détecteur de gaz de référence et en ce que le tronçon intermédiaire (5') de la canalisation de gaz de référence (5) entre les points de raccordement des tronçons de canalisation (7, 8) conduisant au détecteur de gaz de référence (3) peut également être fermé.

3. Détecteur de fuite selon la revendication 2, caractérisé en ce que les deux tronçons de canalisation (7, 8) et le tronçon intermédiaire (5') de la canalisation de gaz de référence (5) sont respectivement munis d'une soupape à 2/2 voies.

4. Détecteur de fuite selon la revendication 2, caractérisé en ce qu'à proximité des points de raccordement des deux tronçons de canalisation (7, 8) à la canalisation de gaz de référence (5) se trouve une soupape à 3/2 voies (16, respectivement 17).

5. Détecteur de fuite selon l'une quelconque des revendications précédentes, caractérisé en ce que les deux tronçons de canalisation (7, 8) à proximité de l'admission (6) du détecteur de gaz de référence (3) présentent des diamètres différents et sont disposés concentriquement l'un par rapport à l'autre.

6. Détecteur de fuite selon la revendication 5, caractérisé en ce que le détecteur de gaz de référence (3) présente une tubulure de raccordement (21) avec une bride (22), en ce que la canalisation de dérivation (7 ou 8) de plus grand diamètre est reliée par l'intermédiaire d'une bride (23) à la tubulure de raccordement (21) et en ce que la canalisation de dérivation (7 ou 8) de plus petit diamètre traverse concentriquement la tubulure de raccordement (21).

7. Détecteur de fuite selon l'une quelconque des revendications précédentes, caractérisé en ce que le détecteur de gaz de référence (3) comprend un spectromètre de masse (14) et une pompe à vide poussé (15) parcourue par le gaz de référence à l'encontre de sa direction de circulation et en ce que la sortie de la pompe à vide poussé (15) constitue l'admission (6) du spectromètre de masse (14).
